# EUROPEAN PATENT APPLICATION

(11) **EP 3 011 964 A1**
(43) Date of publication of application: **27.04.2016**
(21) Application number: 14306696.7
(22) Date of filing: 24.10.2014
(51) Int. Cl.: A61K 35/763, A61K 45/06, A61K 31/7068, A61P 35/00, C12N 7/00

(54) **Compounds and associations for treating pancreatic cancer**

(71) Applicant: Karcinolys, 92500 Rueil Malmaison (FR)
(72) Inventor: Gayral, Marion, 31120 Roques Sur Garonne (FR); Cordelier, Pierre, 31500 Toulouse (FR)
(74) Representative: Cabinet Plasseraud

(57) **Abstract**

A Myb34.5 virus for use in a method of treating pancreatic cancer preferably associated to at least one chemotherapeutic compound, products containing a Myb34.5 virus and at least one chemotherapeutic compound, as a combined preparation for simultaneous, separate or sequential use in therapy of pancreatic ductal adenocarcinoma and pharmaceutical compositions.

## Description

### FIELD OF THE INVENTION

The present invention relates to a Myb34.5 virus and associations of a Myb34.5 virus and of chemotherapeutic compounds for use in a method of treating pancreatic cancer, and to pharmaceutical compositions.

### BACKGROUND OF THE INVENTION

The pancreas is a glandular organ appended to both the digestive and endocrine human systems. The pancreatic endocrine gland produces several important hormones while the exocrine organ secretes enzymes for digestion and absorption of nutrients in the small intestine. Pancreatic cancers can originate from both the endocrine and exocrine glandular systems.

However, tumours derived from the islet cells of the pancreatic endocrine system are a collection of tumour cell types collectively referred to as pancreatic neuroendocrine tumours and not as pancreatic cancer. Their specific histological, phenotypic (hormone secretion) and pathophysiological characteristics, as well as treatment options and prognosis, differentiates them from exocrine pancreatic cancer, referred to as pancreatic cancer.

Pancreatic cancer develops in the exocrine glandular system of the pancreas and is a family of different histological sub-types. The histological classification of tumours of the exocrine pancreas individualises the following types of malignant epithelial tumours: ductal adenocarcinoma, serous cystadenocarcinoma, mucinous cystadenocarcinoma, intraductal papillary-mucinous carcinoma, acinar cell carcinoma, pancreatoblastoma and solid-pseudopapillary blastoma.

Pancreatic cancer is one of the deadliest cancer types. It is the fourth leading cause of cancer deaths and remains one of the most difficult cancers to treat, despite recent moderate therapeutic improvements.

Pancreatic ductal adenocarcinoma (PDAC) is highly resistant to standard treatments. This is a rare disease but since incidence equals mortality, this diagnosis sounds like a death sentence for most cases because of the lack of curative treatment for advanced disease. PDAC has a sophisticated network of biological activities that maintains self-sufficiency in growth signals, is resistant to endogenous antiproliferative signals, evades apoptosis, has limitless replicative potential, and undergoes tissue invasion and metastasis. This heterogeneity stems for the unchallenged resistance of PDAC to conventional therapeutic approaches (chemotherapy, radiotherapy...) and targeted biotherapies.

Since 1997, gemcitabine is the only approved first-line treatment for patients with unresectable locally advanced or metastatic pancreatic cancer. However, the 5-year survival rate is only 2%, with 1-year survival rates ranging from 17 to 23%. Recently, phase II and III trials exploring gemcitabine-based combinations with erlotinib, folfirinox or nab-paclitaxel were found to improve overall survival of patients, leading to the approval of erlotinib and nab-paclitaxel. However, the moderate activity of standard gemcitabine and gemcitabine-based regimens still encourages new therapeutic research programs. An alternative therapeutic approach may rely on viral replication for tumour destruction, by which infection of single tumour cells may induce cell destruction and release of progeny virions that can further spread to the entire tumoral cells population. Indeed, PDAC carcinogenesis is associated with a series of hallmark changes for cancer cells to secure their own growth success. Yet, these very changes (among them lack of interferon response, elevated metabolic activity and disengagement of cell cycle control...) render cancer cells highly sensitive to viral infection.

Several viruses have been explored for their ability to replicate and to inhibit PDAC growth in experimental models. Genetically engineered conditionally replicating herpes simplex virus type 1 (HSV-1) are promising for PDAC therapy because (i) they infect various tumour cell types, (ii) they do not integrate into the genome of infected cells, (iii) they have been shown to be safe in clinical trials, (iv) there are several available anti-HSV-1 specific drugs, such as acyclovir (ACV) to control unwanted infection and (v) total cell killing can be achieved rapidly with a relatively low multiplicity of infection (MOI).

The most widely used strategy to restrict HSV-1 replication to cancer cells is to delete genes that are important for viral replication and to evade innate immune responses, including the IFN-related protein kinase (PKR) antiviral response. *ICP6* encodes the large subunit of viral ribonucleotide reductase (RR), an enzyme involved in *de novo* synthesis of deoxynucleotides. In the absence of viral RR, virus replication depends on host cell RR activity, which has been reported to be higher in cancer cells. Consequently, HSV-1 mutants with deletions in *ICP6* gene preferentially replicate in actively dividing cells such as malignant cells. On the other hand, viral double-stranded RNAs activate various cellular antiviral responses, including PKR, which phosphorylates and inactivates the protein initiation factor eIF-2α thus inhibiting protein synthesis and host cell growth. Amongst other functions that evolved to overcome the innate cellular responses against infection, viral γ₁34.5 interacts with cellular protein phosphatase-1α, resulting in dephosphorylation of eIF-2α thus inhibiting the action of PKR. This allows the re-initiation of protein translation and robust viral replication. Deleting γ₁34.5 may restrict viral cytopathic effect and replication to malignant cells in which eIF-2α phosphorylation is deregulated due to the activation of the K-*Ras* pathway.

However, first-generation mutant HSV-1 viruses with *ICP6, γ₁34.5* or both deletions have demonstrated modest antitumoral activity in PDAC when injected into tumours. Indeed, while *ICP6* deletion can be efficiently complemented by cellular RR, defective *γ₁34.5* mutants display significantly attenuated replication and lysis of cancer cells, when used *in vivo.*

Research is still going on into mutant oncolytic viruses which could be used in patients with advanced PDAC for whom no effective treatment is available.

### SUMMARY OF THE INVENTION

After extensive research, the present inventors found that B*-myb* is over expressed in pancreatic cancer experimental models and primary tumours. They concluded Myb34.5 virus is suitable for targeting to pancreatic cancer-derived cells.

After further research, they found that a second-generation replication-conditional HSV-1 mutant (Myb34.5) in which ICP6 expression is defective and γ₁34.5 expression is driven by the cellular promoter B*-myb* successfully infects and replicates into PDAC derived cells, both *in vitro* and *in vivo* to inhibit cell proliferation and tumour growth.

In addition, they found that Myb34.5 intratumoral injection translates to improved therapeutic efficacy of standard-of-care chemotherapy (gemcitabine).

Myb34.5 is a herpes simplex virus 1 (HSV-1) mutant deleted in the gene for ribonucleotide reductase (ICP6). It also carries a version of γ₁34.5 (a viral gene product that promotes the dephosphorylation of eIF-2α) that is under control of the E2F-responsive cellular B-myb promoter, rather than of its endogenous promoter.

The construction of Myb34.5 virus is for example described by Richard Y. Chung, Yoshinaga Saeki, and E. Antonio Chiocca in J Virol. Sep 1999; 73(9): 7556-7564.

### DESCRIPTION OF THE PREFERRED EMBODIMENTS

Accordingly, the present invention relates to a Myb34.5 virus for use in a method of treating pancreatic cancer, and more particularly in a method of treating pancreatic ductal adenocarcinoma (PDAC).

The present invention also relates to the use of a Myb34.5 virus, for manufacturing a medicament for use in a method of treating pancreatic cancer.

Myb34.5 virus is preferably used in a method of treating pancreatic ductal adenocarcinoma (PDAC).

The present invention further relates to a method of treating pancreatic ductal adenocarcinoma (PDAC) in warm-blooded animals, comprising administering to warm-blooded animals a tumoral cell proliferation inhibiting and tumour growth inhibiting effective amount of at least a Myb34.5 virus.

As previously mentioned, Myb34.5 intratumoral injection provides improved therapeutic efficacy of standard-of-care chemotherapy such as gemcitabine hydrochloride (hereinafter gemcitabine).

Accordingly, the present invention relates to an association of a Myb34.5 virus and a chemotherapeutic compound for use in a method of treating pancreatic cancer, and more particularly in a method of treating pancreatic ductal adenocarcinoma (PDAC).

The present invention therefore also relates to products containing a Myb34.5 virus and a chemotherapeutic compound as a combined preparation for simultaneous, separate or sequential use in therapy of pancreatic cancer, and more particularly of pancreatic ductal adenocarcinoma.

The present invention also relates to the use of an association of a Myb34.5 virus and a chemotherapeutic compound, for manufacturing a medicament for use in a method of treating pancreatic cancer, and more particularly pancreatic ductal adenocarcinoma.

The present invention further relates to a method of treating pancreatic ductal adenocarcinoma (PDAC) in warm-blooded animals, comprising administering to warm-blooded animals a cell proliferation inhibiting and tumour growth inhibiting effective amount of at least a Myb34.5 virus and at least a chemotherapeutic compound.

The association and compounds can be administered preferably parenterally (intravascular such as intravenous or intra-arterial route or intratumoral administration), even though some chemotherapeutic compounds may be administered per os.

The chemotherapeutic compounds are preferably provided in the associations as concentrated solutions for infusion.

The usual daily or weekly dose for Myb34.5 is 10⁵ to 10¹², preferably 10⁶ to 10¹⁰ pfu (plaque forming units) depending on the type and grade of tumour treated and the method of administration for the treatment of pancreatic ductal adenocarcinoma. Preferred dose for Myb34.5 is 10⁶ to 10⁸ pfu once weekly when administered intratumorally or 10⁸ to 10¹⁰ pfu once daily or once weekly when administered intravenously.

The usual daily or weekly dose for the chemotherapeutic compounds depending on the specific compound, the type and grade of tumour treated and the method of administration is for example, 25 to 200 mg per day or 300 to 1.500 mg/m² per week for the treatment of pancreatic ductal adenocarcinoma. For gemcitabine, 500 to 1.000 mg/m2 once weekly for the first 7 weeks, then one week rest, then once weekly for 3 weeks of each 28-day cycle may for example be used. For folfirinox, every other week cycles of oxaliplatin 60 to 85 mg/m², leucovorin 400 mg/m², irinotecan 150 to 180 mg/m² and 5-fluorouracil 400 mg/m² in bolus followed by continuous infusion of 1.050 to 2.400 mg/m² over 46 hours, may for example be used.

The usual daily or weekly dose for Myb34.5 is preferably 10⁶ to 10¹⁰ pfu using gemcitabine or folfirinox as chemotherapeutic compounds.

Preferred usual doses for the association of Myb34.5 and folfirinox as preferred chemotherapeutic compound are, for Myb34.5, 10⁶ to 10⁸ pfu weekly by intratumoral route or 10⁸ to 10¹⁰ pfu daily or weekly by intravenous route, and, for folfirinox, every two weeks, intravenous administration for each cycle of oxaliplatin 85 mg/m², leucovorin 400 mg/m², irinotecan 180 mg/m² and 5-fluorouracil 400 mg/m² in bolus followed by continuous infusion of 2.400 mg/m² over 46 hours.

Preferred usual daily or weekly doses for the association of Myb34.5 and gemcitabine as the most preferred chemotherapeutic compound are, for Myb34.5, 10⁶ to 10⁸ pfu weekly by intratumoral route or 10⁸ to 10¹⁰ pfu daily or weekly by intravenous route and, for gemcitabine, intravenous administration of 1.000 mg/m² once weekly for the first 7 weeks, then one week rest, then once weekly for 3 weeks of each 28-day cycle.

The chemotherapeutic compound and a Myb34.5 virus are most preferably administered separately.

These properties also justify the use of a Myb34.5 virus in a pharmaceutical composition further comprising at least one chemotherapeutic compound.

The novel antitumoral pharmaceutical compositions of the invention are comprised of an effective amount of at least a Myb34.5 virus, an effective amount of a chemotherapeutic compound, and optionally but preferably an inert pharmaceutical carrier or excipient. The compositions may be, for example in the form of tablets, capsules, or injectable solutions or suspensions. Of course one active ingredient may be in one form and another one in another form.

Examples of suitable excipients are aqueous and non-aqueous vehicles, various wetting, dispersing or emulsifying agents and preservatives.

Myb34.5 virus may particularly be formulated for parenteral administration (e.g., by injection, for example bolus injection or continuous infusion). The active ingredient may be presented in unit dose form in ampoules, vials, pre-filled syringes small volume infusion or in multi-dose containers with or without an added preservative. Suitable pharmaceutical forms comprise suspensions, solutions, or emulsions in oily or aqueous vehicles. Examples of oily or non-aqueous carriers, diluents solvents or vehicles include propylene glycol, polyethylene glycol, vegetable oils (e.g., olive oil, and injectable organic esters (e.g., ethyl oleate), and may contain adjuvant agents such as preserving, emulsifying or suspending, wetting, stabilizing and/or dispersing agents. The active ingredients may also be in powder form, obtained by aseptic isolation of sterile solid or by lyophilization from solution for constitution before use with a suitable vehicle, such as sterile, pyrogen-free water.

Among the preferred compositions of the invention are those wherein a Myb34.5 virus is associated with one or more chemotherapeutic compounds, more preferably one or more of the following chemotherapeutic compounds:
- gemcitabine
- folfirinox
- erlotinib or
- nab-paclitaxel,
preferably
- gemcitabine or
- folfirinox
more preferably
- gemcitabine.

Myb34.5 virus is associated with one or more chemotherapeutic compounds in a weight ratio, according to the chemotherapeutic compound used of preferably of (10⁶ to 10¹⁰ pfu/day or week)/(30 to 1.200 mg/m²/week), particularly of (10⁶ to 10¹⁰ pfu/day or week)/(42 to 1.200 mg/m²/week), more particularly of (10⁸ to 10¹⁰ pfu/day or week)/(500 to 1.000 mg/m²/week), and very particularly of approximately (10⁸ pfu/week)/(1.000 mg/m²/week) for intratumoral administration in association with gemcitabine or very particularly of approximately (10¹⁰ pfu/day or week)/(1.000 mg/m²/week) for intravenous administration in association with gemcitabine.

The present invention also relates to a process for preparing a composition described above, characterized in that, according to methods known per se, the chemotherapeutic compound(s) and Myb34.5 virus are mixed with optional acceptable excipients, particularly pharmaceutically acceptable excipients.

Myb34.5 virus and associations and products containing a Myb34.5 virus and at least one chemotherapeutic compound according to the invention have advantageous properties including:
- Myb34.5 virus significantly replicates and kills pancreatic cancer cells.
- Myb34.5 intratumoral injection spares normal adjacent pancreatic tissue but results in the profound inhibition of the progression of an aggressive tumour (massive tumour necrosis, haemorrhage, inflammatory cell infiltrate, scattered tumour cells, induction of apoptosis).
- Combining Myb34.5 and at least one chemotherapeutic compound such as gemcitabine co-treatment provides a very impressive antitumoral effect that is rarely achieved in experimental model of pancreatic cancer.
- Myb34.5 and gemcitabine have synergistic antitumoral activities.
- Myb34.5 administration helps relieving PDAC resistance to conventional chemotherapy.

Therefore, Myb34.5 virus, preferably associated to at least one chemotherapeutic compound preferably selected in the group consisting of folfirinox and gemcitabine, more preferably the latter, may be successfully used in a method of treating pancreatic cancer, and more particularly in a method of treating pancreatic ductal adenocarcinoma.

Myb34.5 is usually provided as a concentrated deep frozen solution which should be thawed and further diluted prior to intratumoral or intravenous injection in patients.

The chemotherapeutic compound such as gemcitabine is usually provided as a powder for solution for infusion which should be further diluted with 0.9% w/v sterile sodium prior to intravenous infusion administration in patients.

The scope of the invention can be understood better by referring to the examples given below, the aim of which is to illustrate and explain the advantages of the invention.

### DESCRIPTION OF THE DRAWINGS

FIG. 1A represents B-myb protein detection in human-derived normal and pancreatic cancer cells. 1: HPNE, 2: HPDE, 3: Capan-1, 4: BxPC-3, 5: Capan-2, 6: Mia PACA-2, 7: Panc-1, 8: negative control (HEK), 9: positive control (Hela).
FIG. 1B represents B-myb RNA expression in patients' tumours vs. normal adjacent tissue.
FIG. 1C represents B-myb protein detection in patients' tumours vs. normal adjacent tissue. Brackets indicate specific signal. B-myb expression levels are indicated below A and C blots.
FIG. 2A is a curve showing Myb34.5 viral titters vs time
FIG. 2B represents cell viability of BxPC-3, Mia PACA-2 and Capan-2 pancreatic cancer-derived human cell lines following infection with Myb34.5.
FIG. 2C represents HSV-1 envelope glycoproteins, caspase-3 and PARP cleavage in Mia PACA-2 cells following infection with Myb34.5.
FIG. 3A and B are curves showing HSV-F and MHG1 viral titters vs time after infection with 0.1 MOI of HSV-F (FIG. 3A) or MGH1 (FIG. 3A) virus.
FIG. 4A and B represent cell viability of BxPC-3, Mia PACA-2 and Capan-2 pancreatic cancer-derived human cell lines after infection with 0.1 MOI of HSV-F (FIG. 3A) or MGH1 (FIG. 3A) virus.
FIG. 5A represents Tumour volume following intratumoral Myb34.5 gene transfer. * p<0.05; ** p<0.01.
FIG. 5B represents the results of haematoxylin and eosin staining of formalin-fixed, paraffin embedded samples of Mia PACA-2 tumours specimens treated by the vehicle or by Myb34.5. Arrows shows inflammatory cell infiltrates.
FIG. 5C and 5D represents the results of Analysis of HSV-1 (5C) or DNA fragmentation (5D) by TUNEL assay.
FIG. 5E represents the results of quantification of DNA fragmentation (expressed as % of apoptotic cells) in each group. * p<0.05; ** p<0.01, difference between placebo and Myb34.5 treated tumours. # p<0.05, difference between tumours treated with 10e8 p.f.u. of Myb34.5 and tumours treated with 10e6 or 10e7 p.f.u. of Myb34.5.

### EXPERIMENTAL DATA

### 1. B-myb expression in pancreatic ductal adenocarcinoma (PDAC) cell lines and tumours.

Myb34.5 is a genetically engineered HSV-1 mutant derived from F strain, defective in *ICP6* gene and that contains the HSV-1 *γ₁34.5* gene under the control of the E2F-responsive, cellular B*-myb* promoter ( Chung RY, Saeki Y, Chiocca EA. B-myb promoter retargeting of herpes simplex virus gamma34.5 gene-mediated virulence toward tumour and cycling cells. J Virol. 1999 Sep;73(9):7556-64).

B*-myb* expression was characterized in normal and tumour pancreatic cell lines by Western blot and quantitative real-time PCR (qPCR). For Western blot, total proteins were extracted from cells or tissues, resolved on SDS-polyacrylamide gels, and transferred to nitrocellulose membrane. After room-temperature blocking for 1 h, blots were incubated overnight at 4°C with antibodies against B*-myb* (Santa Cruz, clone C-20, dilution: 1:500), and GAPD (Santa Cruz, clone 6C5, dilution: 1:1000). Secondary HRP-conjugated antibodies (dilution 1:10000, Perbio Science) were added, and blots were incubated for 1 h at room temperature. Immunoreactive proteins were visualized using Clarity ECL (Biorad) and imaged with ChemiDoc XRS+ (Biorad). For qPCR, total RNA was isolated from tissues with TRIzol Reagent (Life Tech) according to supplier instructions and RNA concentration was measured with the ND-1000 NanoDrop spectrophotometer. B*-myb* mRNA was quantified from 1µg total RNA using the RevertAid First Strand cDNA Synthesis Kit (Thermo Scientific). 18S RNA expression was used as calibrator. Relative amounts were calculated by the comparative cycle threshold (CT) method and expressed as 2^{-ΔCT}, where ΔCT = CT(B*-myb*) *-* CT(18S). Primers used for B*-myb* detection were B*-myb* forward: 5'-TCTGGCTCTTGACATTGTGG-3' SEQ. ID. N°1 and B-*myb* reverse: CGGCAAGGATAGAGACTTGG-3' SEQ. ID. N°2. Duplicate qRT-PCR assays were carried out in a StepOnePlus™ II Real-Time PCR System (Life Tech) with SsoFast EvaGreen supermix (Biorad).

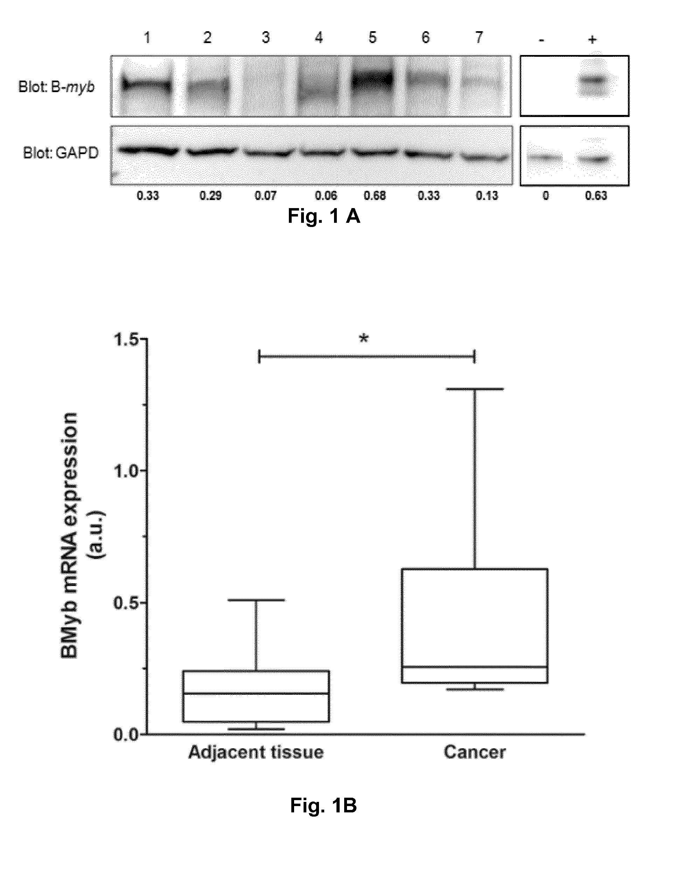

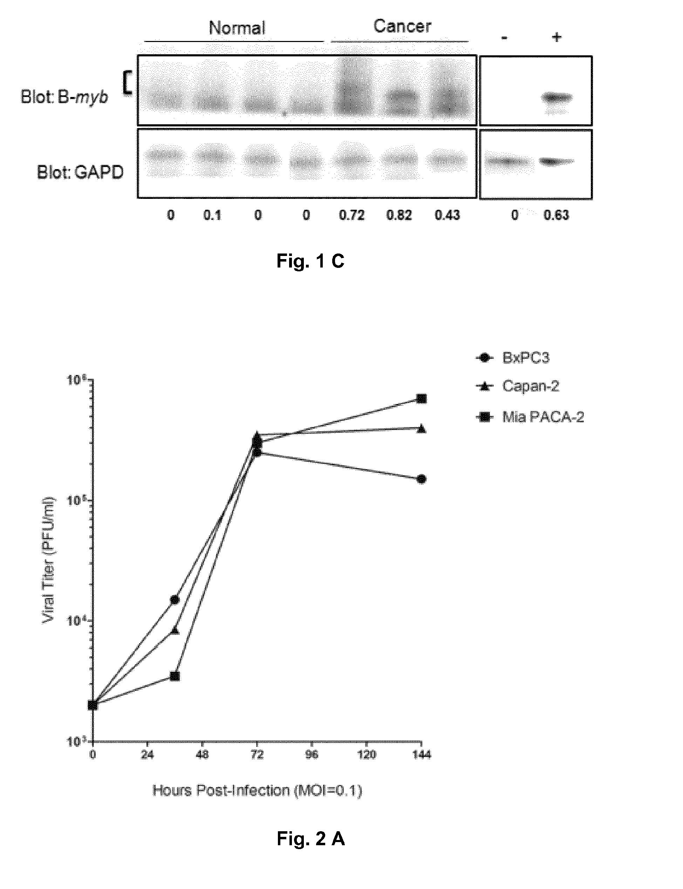

The results are shown in figures 1A, B and C.

In figure 1A, B*-myb* protein is detectable in Capan-2 and Mia PACA-2 pancreatic cancer-derived cell lines, but also in normal HPNE and HPDE pancreatic cell models. On the other hand, B*-myb* is expressed at lower levels in Panc-1, BxPC-3 and Capan-1 pancreatic cancer-derived cell lines (Figure 1A). B*-myb* mRNA is over expressed in 10/12 paired samples of patients' tumours as compared to normal adjacent pancreas (Fig. 1B fold increase 6.1±2.3, p<0.05). This discrepancy of B*-myb* expression between tumour samples and normal adjacent tissue was also detected at the protein level (Fig. 1 C).

Accordingly, B*-myb* is over expressed in pancreatic cancer experimental models and primary tumours and is suitable for Myb34.5 virus targeting to pancreatic cancer-derived cells.

### 2. Replication of Myb34.5 effect of Myb34.5 on PDAC-derived cell lines, in vitro

Since host B*-myb* expression is essential for optimal Myb34.5 replication and oncolytic activity. Accordingly, B*-myb* expression was characterized in PDAC derived cell lines and tumours.

HSV-1 F strain (wild-type HSV-1) was provided by Dr A. Epstein. Recombinant HSV-1 virus Myb34.5 (defective for ICP6 expression; γ₁34.5 expression regulated by the B*-myb* promoter) and MGH1 (defective for both ICP6 and γ₁34.5 expression), both derived from F strain, were kindly provided by E. A. Chiocca (Chairman, department of Neurosurgery, Brigham and Women's Hospital, Harvard Medical School, Boston, MA). Myb34.5, F strain and MGH1 virus were grown and tittered using Vero cells. Human PDAC-derived Capan-2 cells and BxPC-3 were grown in RPMI medium supplemented with 10% fetal calf serum, L-glutamine, antibiotic and antimycotic cocktail (Invitrogen) and Plasmocin® (InvivoGen). Capan-1, Panc-1, MIA PACA-2 were grown in DMEM containing 4.5 g/L glucose (Invitrogen), 10% fetal calf serum, L-glutamine, antibiotics, Fungizone® and Plasmocin® (InvivoGen). All Cell lines were grown in a humidified incubator at 37°C in 5% CO₂.

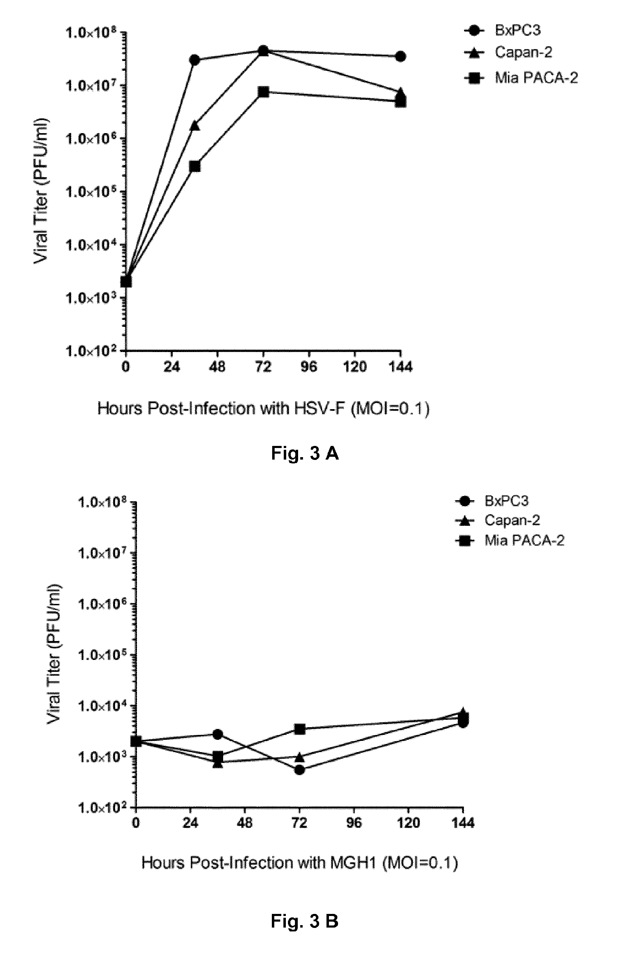

The *in vitro* replication rate of Myb34.5 was measured in PDAC-derived cell lines using multi-step viral recovery experiments. Mia PACA-2, BxPC-3 and Capan-2 cell lines were infected with 0.1 PFU/cell (MOI=0.1) of Myb34.5. Viral replication was determined at 36, 72 and 144 hours post infection. The results are shown in figure 2A. Myb34.5 virus replicates to high level in the three cell lines tested (Figure 2A), while F-strain and MGH1 virus replicated to very high and low levels in PDAC-derived cell lines, respectively (Figures 3A - 3B).

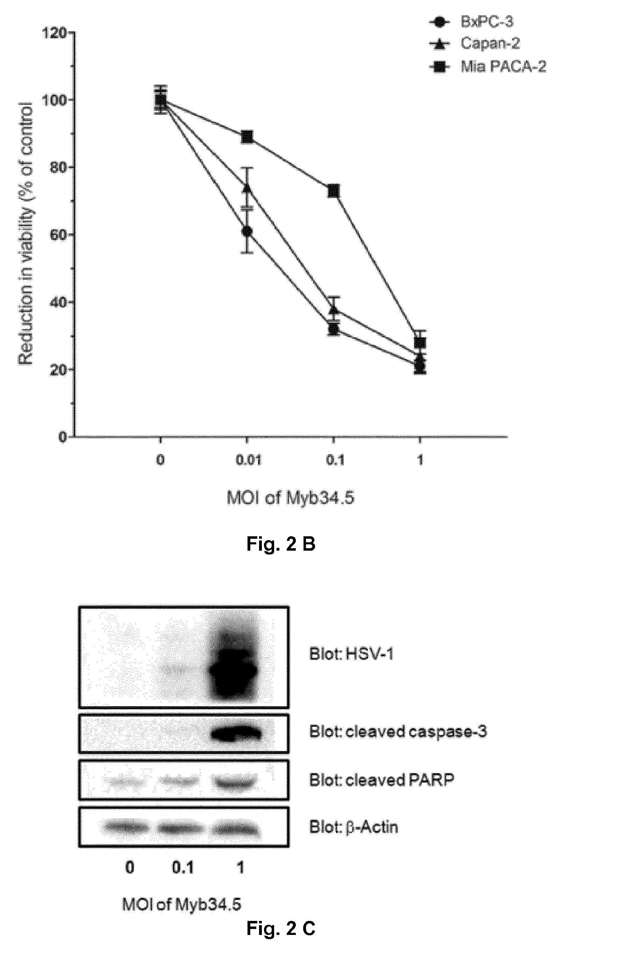

For cytotoxicity assays, human pancreatic cancer-derived cells were seeded at 1×10⁴ cells (Mia PACA-2, Capan-2) or 2×10⁴ cells (BxPC-3) per well in a 96-well dish and grown in complete medium. Twenty four hours later, cells were infected with F strain, MGH1 and Myb34.5 virus at the indicated multiplicities of infection. Six days later, cell viability was determined by fluorometric method using CellTiter-Fluor Cell Viability Assay (Promega) according to manufacturer's instruction. The results are shown in figure 2B. The results show that viability was decreased by Myb34.5 in all cell lines tested; however, Mia PACA-2 cells were more resistant to the cytopathic effect of Myb34.5, as compared to Capan-2 and BxPC-3 cells.

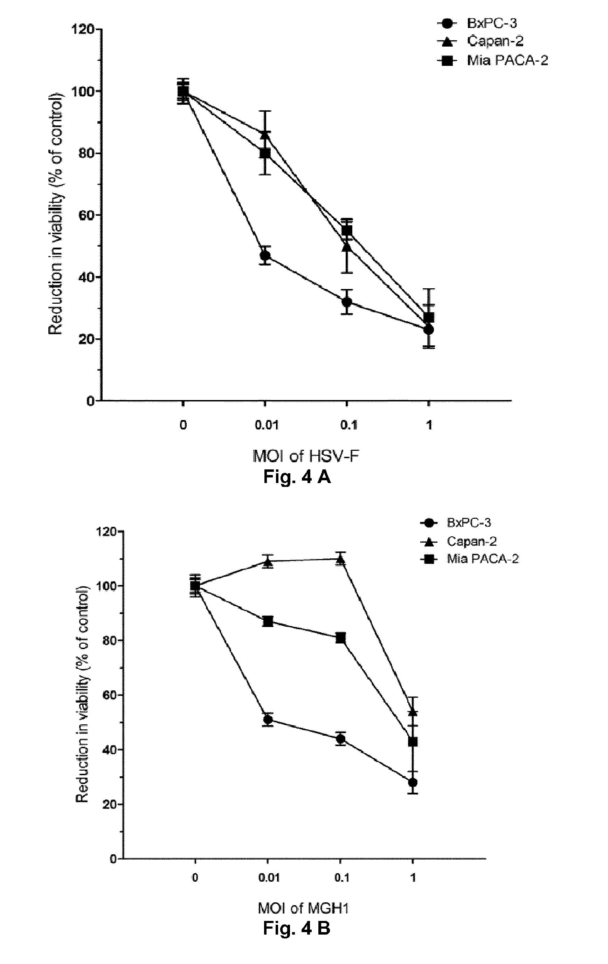

The cytopathic effect of Myb34.5 was consistently greater than that of MGH1 and comparable to HSV-F strain (Figures 4A - 4B). As shown in figure 2C, infection with Myb34.5 resulted in detectable expression of glycoproteins of the viral envelope in PDAC cells (HSV-1 antibody DakoCytomation, clone B0114, dilution: 1:1000). In addition, Myb34.5 dose dependently induced caspase-3 (Cell signalling, dilution 1:500) and PARP (Cell signalling, dilution 1:1000) cleavage in PDAC-derived cell lines, as compared to control-transduced cells.

Accordingly Myb34.5 HSV-1 mutant efficiently replicates in PDAC-derived cell lines, inhibits cell viability and induces apoptosis.

### EXAMPLES

### Example 1: Therapy of PDAC experimental tumours using Myb34.5.

Mia PACA-2 cells were implanted in the tail of pancreas of athymic mice (8 animals per group). Myb34.5 was injected in level 2 animal safety facility in exponentially growing tumours (mean tumour size 150mm³ ± 12mm³) 15 days following tumour induction. Control animals received PBS. Blood samples were collected prior and following Myb34.5 injection. Lucia production was measured in 5µl of serum using coelenterazine (50µM) as a substrate. Tumour volume was calculated using the formula V = (W² × L)/2 were W is width and L is length. Two weeks following treatment, the animals were euthanized and the pancreatic tumours were measured and harvested. Tumours were formalin-fixed, and paraffin embedded and stored at 4°C. H&E staining confirmed the presence of tumour(s) in each pancreas. For Western blot studies, tumours were frozen in liquid nitrogen and stored at -80°C until use.

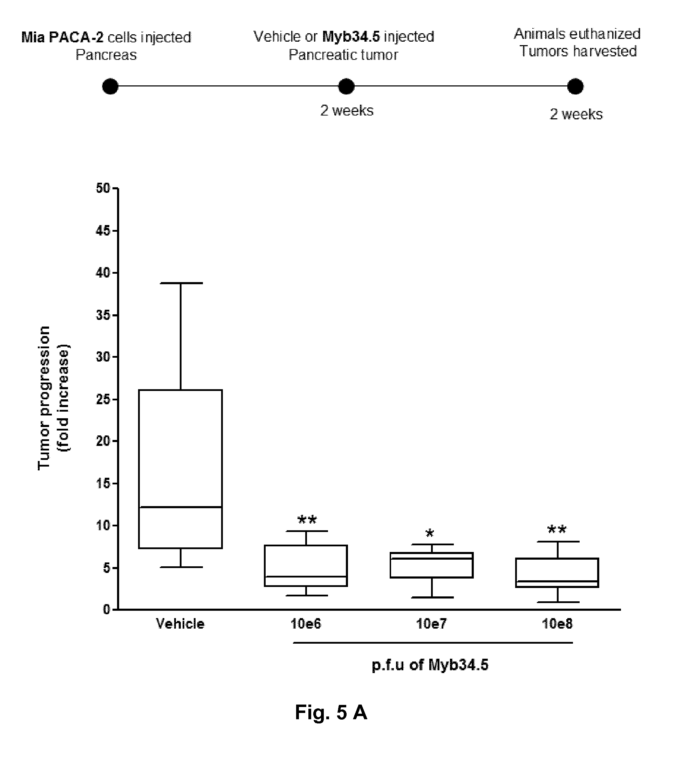

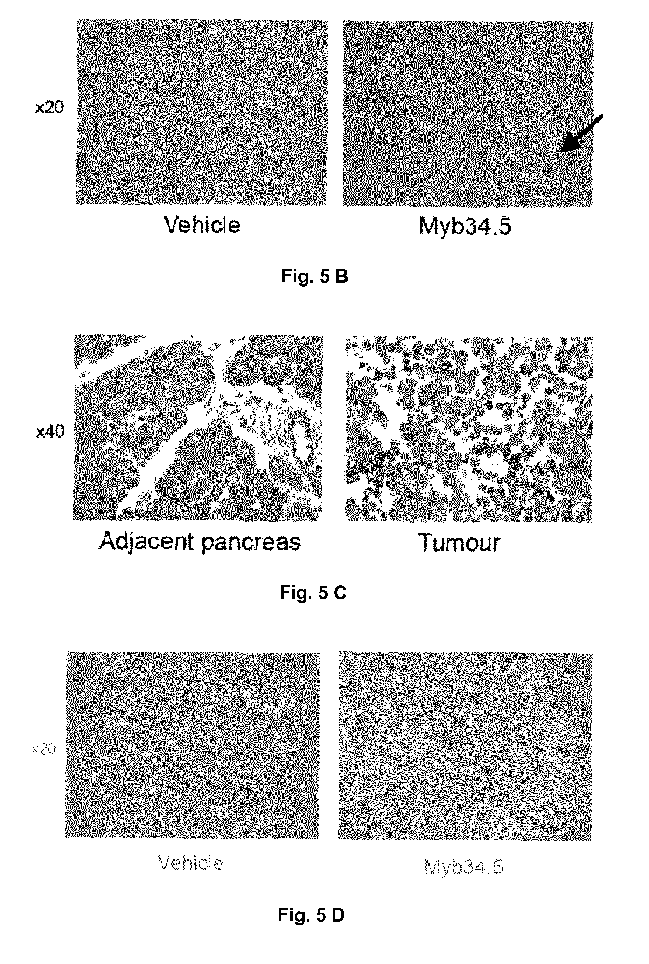

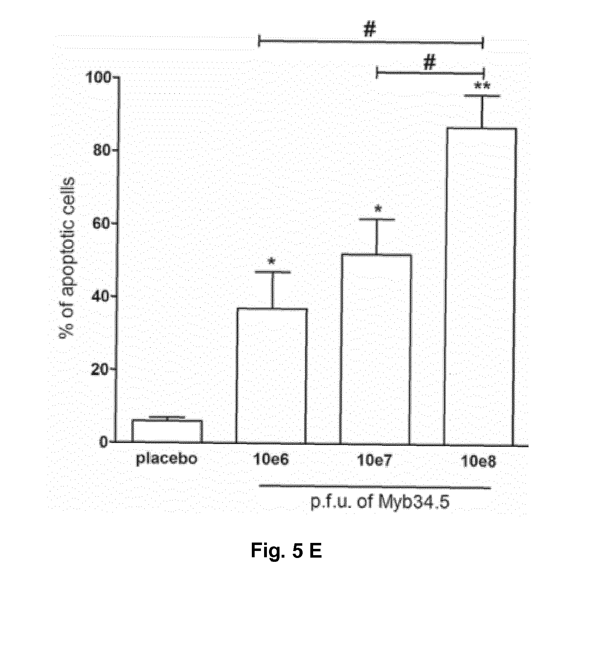

The results are shown in figure 5A and show that Myb34.5 intratumoral injection strongly inhibited tumour progression as tumour burden was reduced by more than 75% with the highest dose of the virus used. The results further show Myb34.5 injection provoked massive tumour necrosis (Fig. 5B), haemorrhage (Fig. 5B, grey area) and inflammatory cell infiltrate (Fig. 5B, arrow), and disorganized tumour cells in comparison to vehicle-treated tumours. By Western blot, HSV-1 proteins were detected in the Myb34.5 treated tumours, but were absent from normal adjacent pancreas (Fig. 5C). Using TUNEL assay (DNA fragmentation performed using *In situ* Apoptosis Detection Kit, Takara Bio Inc., Shiga, JN)., strong induction of apoptosis related to virus doses in tumours treated by Myb34.5 (Fig. 5D and 5E) were observed.

Cumulatively, the above results demonstrate that, *in vivo,* Myb34.5 strictly replicates in tumours, exerts a strong anti-tumour activity against experimental pancreatic cancer, and induces cancer cell death by apoptosis following intratumoral administration.

### Example 2: Combination of Myb34.5 and chemotherapy for PDAC treatment.

Mia PACA-2 Lucia cells were engrafted in the pancreas of athymic mice (6 animals per group). Blood samples were collected prior and following Myb34.5 injection. 10e8 p.f.u. of Myb34.5 virus were administrated with a single intratumoral injection 2 weeks following tumour induction. Control tumours were injected with vehicle.

Gemcitabine hydrochloride (125 mg/kg) was injected *i.p.* every 3 days for 2 weeks. Control animals received 0.9% NaCl *i.p.* Lucia production was measured in 5µl of serum using coelenterazine (50µM) as a substrate.

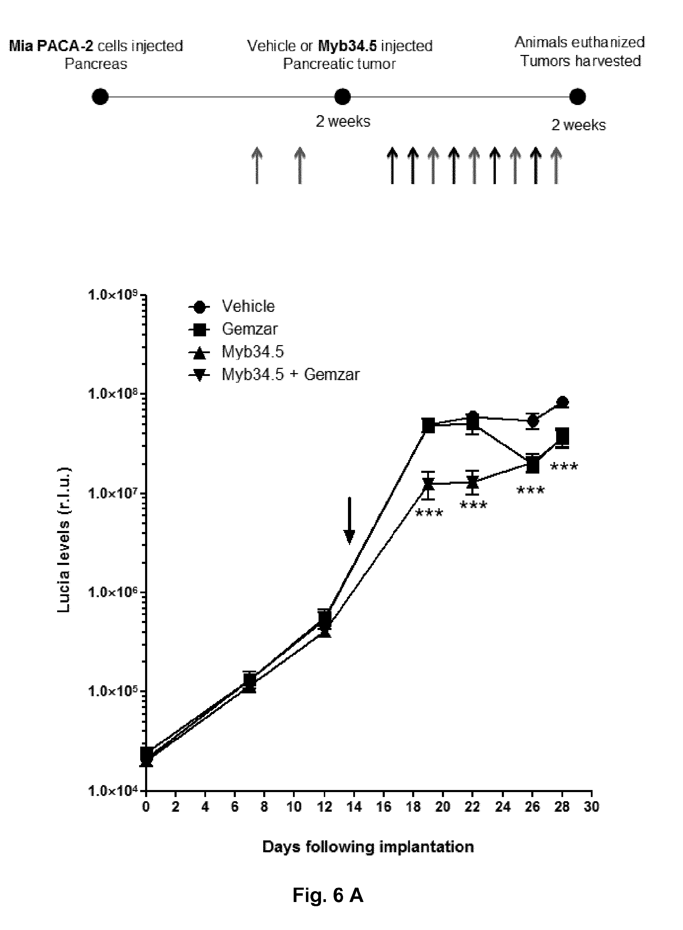

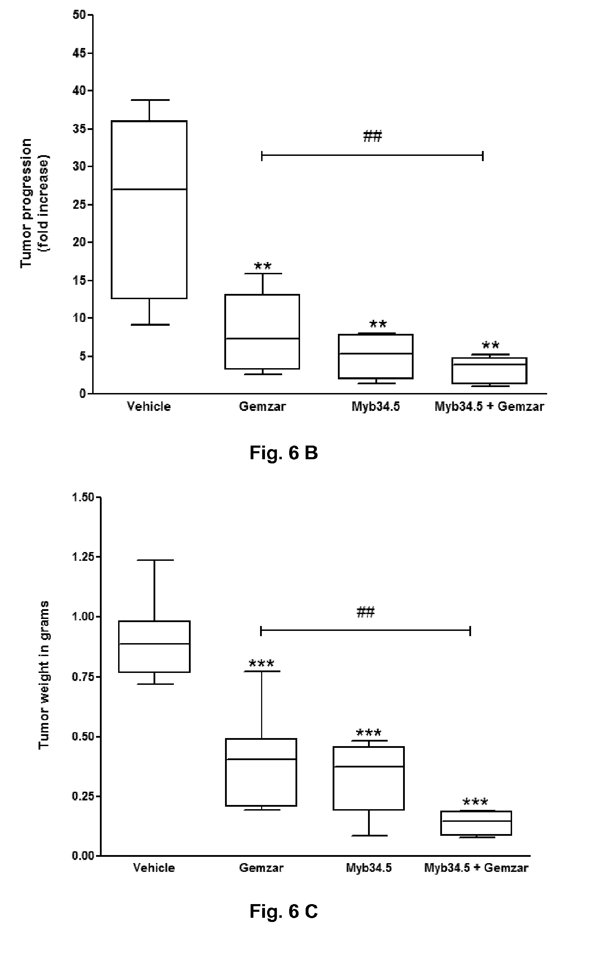

The results are shown in figures 6A, B and C and show that tumour growth was rapidly and significantly inhibited by Myb34.5 injection, and by the combination of gemcitabine hydrochloride treatment and viral infection, while gemcitabine hydrochloride treatment alone resulted in a significant delay in tumour progression inhibition (Fig. 6A). Gemcitabine hydrochloride treatment or Myb34.5 infection alone statistically inhibited tumour growth and tumour weight to similar extent (Fig.6B and Fig.6C). However, combining gemcitabine hydrochloride and Myb34.5 significantly translates to improved therapeutic efficacy of standard-of-care chemotherapy.

Accordingly, the intratumoral injection of Myb34.5 exerts a strong antitumoral effect against very aggressive experimental PDAC tumours. Furthermore, combining targeted HSV-1 viruses with standard-of-care chemotherapy results in a significant higher inhibition of tumour growth.

### Example 3: Combination

1. Myb34.5 virus 10⁸ pfu,
2. Gemcitabine hydrochloride Solution for infusion (Concentrate to be diluted):

### Example 4: Kit

A kit was manufactured comprising in a single packaging
1. a 1 mL vial of 10⁶ pfu/ml Myb34.5 virus
2. a 1.000 mg vial and a 200 mg vial of gemcitabine hydrochloride in powder form Excipients: Mannitol, Sodium acetate, Hydrochloric acid and Sodium hydroxide.

### Example 5: Kit

A kit was manufactured comprising in a single packaging
1. a 1 mL vial of 10⁸ pfu/ml Myb34.5 virus
2. a 1.000 mg vial and a 200 mg vial of gemcitabine hydrochloride in powder form; Excipients: Mannitol, Sodium acetate, Hydrochloric acid and Sodium hydroxide.

## Claims

1. A Myb34.5 virus for use in a method of treating pancreatic cancer.

2. The Myb34.5 virus according to claim 1, **characterized in that** pancreatic cancer is pancreatic ductal adenocarcinoma (PDAC).

3. An association of a Myb34.5 virus and of at least one chemotherapeutic compound, for use in a method of treating pancreatic cancer.

4. An association according to claim 3, **characterized in that** pancreatic cancer is pancreatic ductal adenocarcinoma.

5. Products containing a Myb34.5 virus and at least one chemotherapeutic compound, as a combined preparation for simultaneous, separate or sequential use in therapy of pancreatic ductal adenocarcinoma.

6. Products according to claim 5, **characterized in that** the at least one chemotherapeutic compound is selected in the group consisting of gemcitabine and folfirinox.

7. Products according to claim 5, **characterized in that** the at least one chemotherapeutic compound is provided as a concentrate for solution for infusion.

8. A pharmaceutical composition comprising a Myb34.5 virus and at least one chemotherapeutic compound.

9. A pharmaceutical composition according to claim 8, **characterized in that** the at least one chemotherapeutic compound is selected in the group consisting of gemcitabine and folfirinox.

10. A pharmaceutical composition according to claim 8 or 9 adapted to parenteral administration.


SEQUENCE LISTING

<110>  KARCINOLYS

<120>  Compounds and associations for treating pancreatic cancer

<130>  BEP140523 EP

<160>  2

<170>  PatentIn version 3.5

<210>  1
<211>  20
<212>  DNA
<213>  artificial

<220>
<223>  primer

<400>  1
tctggctctt gacattgtgg                                                   20


<210>  2
<211>  20
<212>  DNA
<213>  artificial

<220>
<223>  primer

<400>  2
cggcaaggat agagacttgg                                                   20